# EUROPEAN PATENT APPLICATION

(11) **EP 1 111 388 A1**
(43) Date of publication of application: **27.06.2001**
(21) Application number: 99870283.1
(22) Date of filing: 23.12.1999
(51) Int. Cl.: G01N 33/576, A61K 38/16, A61K 38/17

(54) **Interaction between CD14 and HBV components**

(71) Applicant: UNIVERSITEIT GENT, 9000 Gent (BE)
(72) Inventor: Leroux-Roels, Geert, 9000 Gent (BE); Vanlandschoot, Peter, 9000 Gent (BE)
(74) Representative: De Clercq, Ann

(57) **Abstract**

The present invention is based on the finding that CD14 is a receptor for HBV. The invention more particularly describes molecules having HBV receptor activity. The invention also relates to new compounds directed against HBV infections, and methods for identifying them, including in vitro and in vivo model systems to do so. The invention further relates to new vaccine compositions directed against HBV. Additionally the invention also relates to the use of HBV components to treat inflammatory diseases.

## Description

The present invention relates to the field of Hepatitis B virus (HBV) infections. The invention describes molecules having HBV receptor activity. The invention also relates to new compositions directed against HBV infection, as well as to new vaccine compositions directed against HBV. Additionally the invention also relates to the use of HBV components to treat inflammatory diseases.

Worldwide the Hepatitis B virus (HBV) causes more then 1 million deaths per year and about 350 million people are persistently infected with the virus. Most adult individuals will clear a primary infection, which can be asymptotic or can result in acute liver injury. However, approximately 5% will not resolve the primary infection and go on to a persistent infection. Vaccination is an effective way to prevent infection, but the giant virus reservoir in persistent carriers is a major obstacle to rapid eradication of the virus. The mechanism by which HBV establishes this persistent state is at present still unclear, but a failing immune response is clearly involved. In patients suffering from an acute resolving HBV infection strong and multispecific CTL responses towards envelope proteins are observed, whereas T helper cell and B cell responses against the envelope is less pronounced. In these patients there is a strong T helper cell response towards the capsidprotein (HBcAg) as well as a B cell response (Milich, 1997). In chronic HBV patients no or very weak pauci-specific responses towards envelope and core proteins are measured. A humoral response towards HbcAg is always discernible (Milich, 1997). In vitro studies have demonstrated a reduced capacity of PBMC from chronic infected persons to produce cytokines (Muller & Zielinski, 1990; Muller & Zielinski, 1992; Nagaraju et al., 1998; Vingerhoets et al., 1998). Administration of cytokines or cytokine induced by another (non- HBV) viral infection has a strong antiviral effect in mouse models of HBV infection (Cavanaugh et al. 1998)

The mechanism by which HBV enters cells has also not been elucidated. To infect a cell HBV must first bind to a host cell receptor via one or a combination of the three related viral membrane proteins L, M and S, which share 226 carboxy terminal amino acids. The M protein contains an additional pre-S2 (55 amino acids) sequence. The L protein contains S, pre-S2 and a pre-S1 sequence (108-119 amino acids). These membrane proteins are also found in the non-infectious surface antigen particles (HBsAg), which are present in circulation. Like virions such particles contain predominantly the S protein, but smaller amounts of M and hardly any L protein (Gerlich and Bruss, 1993).

Recombinant expression of only the S protein results in assembly and secretion of non-infectious surface antigen particles. Such viral like particles (VLP's) have been used widely to identify cellular receptors. Initially the preS2 region was considered to be involved in the attachment process. Binding to polymerized human serum albumin, an unusual glycan structure, fibronectin and the transferrin receptor were reported. In recent years it has been suggested that the preS1 domain contains the attachment site. Binding to glyceraldehyde-3-phosphate-dehydrogenase, an IgA receptor, interleukin 6 and asialoglycoprotein were demonstrated. Attachment of the S protein to annexin V and apolipoptotein H has also been observed. (Imai et al., 1979; Franco et al., 1992; Gerlich et al., 1993; Budkowska et al., 1995; Petit et al., 1992; Pontisso et al., 1992; Neurath et al., 1992; Treichel et al., 1994; Hertogs et al., 1993; Mehdi et al., 1994).

Hepatocytes are believed to be the major natural host cells for Hepatitis B virus (HBV), but viral DNA and viral proteins have been detected in many other tissues and cell types. (Blum et al., 1983; Dejean et al., 1984; Zeldis et al., 1986; Harrison et al., 1990; Neurath et al., 1990; Mason et al., 1993).

It has been postulated that cells of the lymphoid system may initially support replication (Korba et al., 1989). Keeping in mind the principal routes of HBV transmission (via blood contacts and mucosal surfaces), it seems not unlikely that like for HIV, the first cells infected by HBV are indeed monocytes/macrophages or T and B cells. Infection implies the presence of HBV specific receptors on the membrane of these cells. Therefore the possible interaction of HBsAg with PBMC was investigated by fluorescence-activated cell sorting (FACS), using biotinylated recombinant HBsAg particles, which only contain S protein (b-rHBsAg). It is reported here that such particles bind almost solely to monocytes and not to T-cells, while some recognition of B-cells is observed.

The aim of the present invention is to provide having HBV receptor activity and their use.

Another aim of the invention is to provide compositions for preventing, treating or alleviating HBV infections.

Another aim of the invention is to provide new vaccine compositions for preventing, treating or alleviating HBV infections.

Another aim of the invention is to provide compositions for preventing treating or alleviating inflammatory reactions.

Another aim of the invention is to provide screening processes for identifying compounds interfering with HBV components.

It is another aim of the invention to provide methods for preparing compositions including the compounds of the invention.

It is a further aim of the invention to provide cellular hosts to be used as a model system for testing potential drugs against HBV infection and to provide for assays which involve the use of this host.

Another aim of the present invention is to provide mammalian non-human transgenic animals with symptoms related to HBV, and for the use of these transgenic animals as a model system for testing potential anti-HBV drugs.

All the aims of the present invention have been met by the embodiments as set out below.

Although the liver is the major target organ for HBV other cells may become infected. In PBMC highest infection rates were detected in monocytes, followed by B-cells and T-cells (Pontisso et al., 1991; Trippler et al., 1999). Data presented in the present invention clearly demonstrate that HBsAg particles, which contain only the S protein, specifically bind to monocytes through interaction with CD14 molecules. Expression of CD14 by human hepatocytes has recently been demonstrated (Hetherington et al., 1999; Su et al., 1999). This raises the possibility that HBV can attach to hepatocytes using the same receptor. The present inventors were able to isolate and characterise the HBV receptor present on monocytes as being CD14.

Consequently, in a first embodiment, the present invention relates to a method for determining the binding between CD14 and Hepatitis B virus components comprising the use of:
a) CD14, or a mutein, or a fragment of CD14 which is able to bind to HBV antigens or particles,
b) a ligand of the molecules mentioned in a),
c) an antibody raised against the molecules mentioned in a),
d) an antibody raised against the ligand mentioned in b),
e) an anti-idiotype antibody raised against the antibody mentioned in c),
f) an anti-idiotype antibody raised against the antibody menitioned in d)
g) HBV components, or,
h) antibodies raised against the molecules or particles mentioned in g).

Human CD14 (hCD14) is predominantly expressed on cells of the myelomonocytic lineage including monocytes, macrophages and Langerhans cells (Goyert et al., 1986; Ferrero et al., 1990). hCD14 is also expressed at lower levels on neutrophils and B cells (Labeta et al., 1991). Recently CD14 expression by human hepatocytes has been demonstrated (Hetherington et al., 1999; Su et al., 1999).The mature protein contains 356 amino acids; a signal sequence of 19 aa is removed during processing. The molecular weight of the polypeptide is 35773, while on SDS-PAGE it is 53-55 kDa. CD14 is anchored to the cell membrane by linkage to glycosylphosphatidylinositol (GPI-anchor; Haziot et al., 1988) and contains 4 N-linked carbohydrates. O-linked glycosylations are present as well (Stelter et al., 1996). At least two soluble forms of CD14 (sCD14) have been described. One is produced by shedding of CD14 expressed on the cell membrane. A second form is released from cells before addition of the GPI anchor (Bufler et al., 1995). Several mAbs, like My4 and M(P9), directed against CD14 and having different characteristics have been isolated and described. Many of these are commercially available. The recognition of CD14 by such mAbs may differ from cell type to cell type, although CD14 mRNA from different cell types show identical nucleotide sequences. Mouse (mCD14) has been sequenced and shows less then 70% homology with hCD14 (Setoguchi et al., 1989;Matsuura et al., 1989; Ferrero et al., 1990). Tissue distribution of mCD14 is different when compared to hCD14 (Fearns et al., 1995)._mCD14 for example is expressed on peritoneal monocytes but not on blood monocytes.

In the present invention, the term "CD14" relates to the full-length polypeptide and may be from human or any non-human mammalian origin.

The term "mutein derived from CD14" which is able to bind to HBV components refers to a derivative or homologue of naturally occuring CD14 which may be experimentally indentified to bind as well as CD14 to HBV components under the following conditions. Wells of an ELISA plate are coated with the purified mutein of CD14 (1 µg/ml in PBS). After blocking with 0.1% BSA in PBS, biotinylated HBsAg (1 µg/ml in PBS-0.1% BSA) is added to the wells. After incubation for 1.5 h the wells are washed and are incubated with alkaline phophatase-conjugated streptavidine. After washing, substrate solution (1mg/ml Na p-nitrophenyl in diethylamine buffer pH 9.8) is added. The absorbance is measured at 405 nm at appropriate times.

The term "fragment of CD14" which is able to bind to HBV components refers to a shorter version of CD14 or a mutein thereof and may be experimentally indentified to bind as well as CD14 to HBV components under the following conditions. Wells of an ELISA plate are coated with the purified shorter version of CD14 (1 µg/ml in PBS). After blocking with 0.1% BSA in PBS, biotinylated HBsAg (1 µg/ml in PBS-0.1% BSA) is added to the wells. After incubation for 1.5 h the wells are washed and are incubated with alkaline phophatase-conjugated streptavidine. After washing, substrate solution (1mg/ml Na p-nitrophenyl in diethylamine buffer pH 9.8) is added. The absorbance is measured at 405 nm at appropriate times.

It is to be understood that in the context of the present invention the term "ligand" means "receptor ligand" and refers to any ligand which is able to bind to CD14 in a specific way. It is capable of competitively inhibiting the binding of biotinylated HBsAg to CD14. The property of binding to CD14 can be tested using the BlAcore system in which the binding of the ligand to adsorbed CD14 is monitored (Malmqvist, 1999).

It is to be noted that the above-mentioned binding conditions are given as an example, and can be modified by the person skilled in he art within limits such that the function of binding remains equivalent to the one defined under the above-mentioned conditions.

Preferred illustrations of ligands include for instance antisense peptides as defined below, as well as possible drugs which specifically bind to CD14 present on the cell's plasma membranes.

The term "antisense peptide" is reviewed by Blalock (1990) and by Roubos (1990). In this respect, the molecular recognition theory (Blalock, 1990) states that not only the complementary nucleic acid sequences interact but that, in addition, interacting sites in proteins are composed of complementary amino acid sequences (sense-receptor ligand or sense-antisense peptides). Thus, two peptides derived from complementary nucleic acid sequences in the same reading frame will show a total interchange of their hydrophobic and hydrophilic amino acids when the amino terminus of one is aligned with the carboxy terminus of the other. This inverted hydropathic pattern might allow two such peptides to assume complementary conformations responsible for specific interaction.

The expression "anti-idiotype antibodies raised against the antibody mentioned in c)" refers to monoclonal antibodies raised against the antigenic determinants of the variable region of the monoclonal antibodies raised against CD14, or a mutein, or a fragment of CD14 which is able to bind to HBV antigens. These antigenic determinants of immunoglobulins are known as idiotypes (sets of idiotypes) and can therefore be considered to be the "fingerprint" of an antibody (for review see de Préal, 1978; Fleishmann et Davie, 1984). The mehods for production of monoclonal anti-idiotypic antibodies have been described by Gheuens and MacFarlin (1982). Monoclonal anti-idiotypic antibodies have the property of forming an immunological complex with the idiotype of the monoclonal antibody against which they were raised. In this respect the monoclonal antibody is referred to as Ab1, and the anti-idiotypic antibody is referred to as Ab2. Ab2 to CD14, or to muteins or fragments derived thereof can thus recognise the receptor binding site on HBV, and can consequently block this HBV binding site, thereby preventing HBV attachment and infection.

On the other hand, the expression "anti-idiotype antibodies raised against the antibody mentioned in d)" refers to monoclonal antibodies raised against the antigenic determinants of the variable region of the monoclonal antibodies raised against a ligand or antisense peptide of the molecules described in a). In this respect the monoclonal antibody is referred to as Ab3, and the anti-idiotypic antibody is referred to as Ab4. Ab4 to ligand or antisense peptides of the molecules of a) can thus recognize the ligand or antisense peptide binding site on CD14 and can consequently block this binding site, thereby preventing or interfering with HBV attachment and infection.

The anti-idiotypic antibodies (Ab2 and Ab4 respectively) may themselves be used as antigens to produce anti-anti-idiotypic antibodies (Ab5 and Ab6 respectively) to these polypeptides, or to the ligand or antisense peptides respectively, which may serve as substitutes for Ab1 or Ab3 respectively.

The expression "anti-anti-idiotype antibodies against CD14" refers to antibodies raised against the variable region of anti-idiotype antibodies against CD14. Analogous, the expression "anti-anti-idiotype antibodies against the ligand or antisense peptides" refers to antibodies raised against the variable region of anti-idiotype antibodies against against the ligand or antisense peptides.

The monoclonal antibodies according to the invention are preferably human monoclonal antibodies or humanized versions of monoclonal antibodies.

In the present invention, the term Hepatitis B virus (HBV) "components" refers to the HBV virus, HBV molecules, particles, antigens, peptides or more specific fragments of the Hepatitis B viral or envelope polypeptides.

The term " HBV particles" refers to non-infectious surface antigen particles (HBsAg) containing the viral membrane proteins and are found in the circulation. HBsAg is a macromolecular particle that is composed of protein, carbohydrates (in the form of glycoproteins) and lipids. The lipid moiety is of host origin and represents approximately 25% of the particle mass. Like virions such particles contain predominantly the S protein, but smaller amounts of M and hardly any L protein. Frequently used HBV particles are the recombinant non-infectious surface antigen particles (rHBsAg) produced in *S. cerevisiae* and which are commercially available by Smithkline Beecham Biologicals (Rixensart, Belgium) and Merck Sharpe and Dohme (MSD, USA). rHBsAg particles contain only the S protein.

The expression "determining the binding" in claim 1 refers to all methods known in the art to possibly assess the binding between CD14 and HBV components. For instance "determining the binding" may be experimentally defined under the following. Wells of an ELISA plate are coated with purified CD14 (1 µg/ml in PBS). After blocking with 0.1% BSA in PBS, biotinylated HBsAg (1 µg/ml in PBS) is added to the wells. After incubation for 1.5 h the wells are washed and are incubated with alkaline phophatase-conjugated streptavidine. After washing, substrate solution (1mg/ml Na p-nitrophenyl in diethylamine buffer) is added. The absorbance is measured at 405 nm at appropriate.

The expression "the use of" in claim 1 refers to any use in any method known to the one skilled in the art. This can be for instance the use in binding assays, ELISA's, FACS analysis, Western Blots, Line immuno-assay (Lia), BIAcore real time detection system.

The invention also relates to a composition for preventing, treating or alleviating HBV infections comprising as an active substance at least one of the following :
a) CD14 or a mutein or fragment thereof which binds to HBV components,
b) a ligand or antisense peptide of the molecules mentioned in a),
c) an antibody raised against the molecules mentioned in a),
d) an antibody raised against the ligand or antisense peptide mentioned in b),
e) an anti-idiotype antibody raised against the antibody mentioned in c),
f) an anti-idiotype antibody raised against the antibody menioned in d),
g) fragments of HBV envelope proteins which bind to CD14, and,
h) antibodies specifically recognizing the fragments mentioned in g).

The composition according to the invention can be a pharmaceutical, diagnostic or vaccine composition.

According to the invention, the peptides or polypeptides referred to by a), the antibodies referred to by d) and the anti-idiotype antibodies referred to by e) in claim 2, compete with CD14 for the CD14-binding site on HBV or on HBV components. These polypeptides, muteins and fragments, monoclonal or polyclonal antibodies and anti-idiotype antibodies are (pharmacologically) active insofar as they prevent virus attachment and infection or prevent HBV components from attaching to the cells.

According to the invention, the ligand or antisense peptides polypeptides referred to by b), the antibodies referred to by c) and the anti-idiotype antibodies referred to by f) in claim 2 have the properties of competing with the virus or with the HBV components for the HBV binding site present on CD14. These ligand or antisense peptides, monoclonal or polyclonal antibodies and anti-idiotype antibodies are (pharmacologically) active insofar as they block the Hepatitis B virus or the HBV component binding site on the CD14 molecule(s) present on the cell membrane and thus protect the cell from virus attachment and infection or HBV component attachment.

According to the invention the anti-idiotype antibodies referred to by e) in claim 2, recognize the receptor-binding site on HBV or on HBV components and can consequently block this binding site of the virus or the HBV component, thereby preventing virus attachment and infection or HBV component attachment.

Analogous, anti-idiotype antibodies referred to by f) in claim 2, are competitors with the virus or the HBV components and are (pharmacologically) active insofar as they block the HBV binding site of CD14 on the cell and protect the cell from attachment of the virus or HBV components.

The expression "the HBV binding site" more specifically relates to the HBsAg or/and rHBsAg binding site.

The expression "fragments of HBV envelope proteins" relates to shorter versions of the HBV envelope proteins insofar as they contain the CD14 binding site. In a preferred embodiment, they contain the antigenic determinants recognized by antibodies. These antibodies may specifically interfere with the binding between CD14 and HBV and thus prevent virus attachment and infection or prevent HBV components from attaching to the cells.

The invention also relates to vaccine compositions for preventing, treating or alleviating HBV infections comprising as an active substance at least one of the following:
a) receptor ligands or antisense peptides as defined in claim 1 or antibodies directed against these ligands or antisense peptides,
b) anti-idiotype antibodies raised against the receptor ligands or antisense peptides as defined in claim 1,
c) muteins or fragments of CD14 as defined in claim 1 or antibodies directed against these muteins or fragments,
d) immunogenic fragments of HBV antigens which interact with CD14 fragments comprising epitopes of antigens of HBV which bind to CD14, or,
e) antibodies specifically recognizing the fragments or d).

The term "immunogenic fragments of HBV antigens" refers to fragments which are able to raise antibodies against the HBV antigens.

CD14 has been shown to be involved in many immune processes. First of all, CD14 is the major LPS receptor (Wright et al., 1990), but binds also to other microbial products (Newman et al., 1995; Soell et al., 1995; Heumann et al., 1994; Kusunoki et al., 1995). These interactions provoke the release of cytokines (TNFα, IL-1 and IL-6), which trigger acute phase responses (Moshage, 1997). However, engagement of CD14 does not necessarily result in production of these cytokines. It has recently been shown that monocytes phagocytose apoptotic T-cells through interaction with CD14 without release of inflammatory cytokines (Devitt et al., 1998). Recent work also suggest that this interaction generates signals that actively suppress pro-inflammatory signals (Voll, 1997; Fadok et al., 1998). CD14 is also supposed to be involved in the regulation of monocyte apoptosis, because increased or decreased expression precedes survival or apoptosis, respectively (Heidenreich et al., 1997). Monocyte and T- and B cell activation is probably also dependent on interactions of CD14 with unknown receptors. Engagement of CD14 by specific mAbs on monocytes results in suppression of LPS and II-2 induced monocyte responses (Bosco et al., 1997), terminates T-cell proliferation and inhibits synthesis of IgG's (Lue et al., 1991; Jabara et al., 1994).

The present inventors were able to show that HBsAg inhibits LPS induced activation of monocytes as demonstrated by the reduced secretion of pro-inflammatory cytokines II-1β and TNFα. This anti-inflammatory potential could be involved in the establishment of a persistent virus infection.

These observations raise the possibility that
a) preventing the attachment of HBsAg to CD14 in infected patients will result in the release of inflammatory cytokines. These cytokines could help to clear the infection,
b) a HBV derived vaccine which does not bind to CD14 is more likely to be more immunogenic,
c) because of the anti-inflammatory potential of HBsAg these particles can be used as a therapeutic agent for treating inflammatory reactions and endotoxine-induced responses (shock).

The invention thus relates to a composition for treating, preventing or alleviating inflammatory reactions in patients comprising the use of HBV components.

In the alternative, the invention relates to the use of HBV components for the manufacture of a medicament for treating, preventing or alleviating inflammatory reactions.

The HBV vaccine compositions according to the present invention which contain either muteins or fragments of CD14 as defined above or ligand or antisense peptides or anti-idiotypic antibodies against the ligand and antisense peptides or immunogenic fragments of HBV antigens or antibodies against said fragments, have certain advantages over the already known HBV vaccine compositions, because they are more likely to give rise to an immune response in those HBV infected patients who would not respond to the already existing HBV vaccine compositions.

Vaccine compositions of the prior art are for instance, described in Ellis, R.W, 1993 and further in Blumberg, 1997; Papaevangelou, 1998; Adkins and Wagstaff, 1998.

Vaccine compositions may be used either prophylactic or therapeutic.

The polypeptides of the present invention may be produced by recombinant expression in prokaryotic and eukaryotic host cells such as bacteria, yeast, fungi or mammalian cells, which have been previously transformed by a recombinant vector coding for the polypeptides of the invention. It is expected that those of skill in the art are knowledgeable in the numerous expression systems available for expression in these systems.

A "recombinant" vector will comprise a recombinant DNA molecule comprising a "heterologous sequence" meaning that said recombinant DNA molecule will comprise a sequence originating from a foreign species, or, if from the same species, may be substantially modified from its original form. For example, a promoter operably linked to a structural gene which is from a species different from which the structural gene was derived, or, if from the same species, may be substantially modified from its original form.

It should be understood that the "heterologous sequence" can be the complete CD14 gene or nucleic acid sequences encoding muteins or fragments or peptides derived from CD14 and may be from human or any non-human mammal origin. The "heterologous sequence" can also be a nucleic acid sequence coding for HBV components, or fragments thereof or peptides derived thereof.

The polypeptides and peptides of the invention can also be prepared by classical chemical synthesis.

The synthesis can be carried out in homogeneous solution or in solid phase. For instance, the synthesis technique in homogeneous solutuion which can be used is the one described by Houbenweyl in the book entitled "Methode der organischen chemie" (Method of organic chemistry) edited by E. Wunsh, vol. 15-1 et II, THIEME, Stuttgart 1974.

The polypeptides of the invention can also be prepared in solid phase according to the methods described by Atherton and Shepard in their book entitled "Solid phase peptide synthesis" (IRL Press, Oxford, 1989).

All these methods are well known to those skilled in the art.

The antisense peptides can be prepared as described in Ghiso et al. (1990). By means of this technology it is possible to logically construct a peptide having a physiological relevant interaction with a known peptide by simple nucleotide sequence analysis for complementarity, and synthesize the peptide complementary to the binding site.

The monoclonal antibodies of the invention can be produced by any hybridoma able to be formed according to classical methods from splenic lymphocytes of an animal, particularly of a mouse or rat, immunized against the CD14 or muteins thereof or ligand or antisense peptides defined above, on the one hand, and of cells of a myeloma cell line on the other hand, and to be selected by the ability of the hybridoma to produce the monoclonal antibodies recognizing the polypeptides which has been initially used for the immunization of the animals.

The antibodies involved in the invention can be labeled by an appropriate label of the enzymatic, fluorescent or radioactive type or any label known in the art.

The monoclonal antibodies according to this embodiment of the invention may be humanized versions of mouse monoclonal antibodies made by means of recombinant DNA technology, departing from parts of mouse and/or human genomic DNA sequences coding for H and L chains or from cDNA coding for H and L chains.

Alternatively the monoclonal antibodies according to this embodiment of the invention may be human monoclonal antibodies. These antibodies according to this embodiment of the invention can also be derived from human peripheral blood lymphocytes (PBL) of patients infected with HBV, or vaccinated against HBV. Such human monoclonal antibodies are prepared, for instance, by means of human peripheral blood lymphocytes repolution of severe combined immune deficiency (SCID) mice (for review, see Duchosal et al. 1992)

The anti-idiotype antibodies raised against CD14, or against its muteins, or against the fragments as defined above, or anti-anti-idiotype antibodies raised against CD14 as defined above can be prepared as mentioned above.

The anti-idiotype antibodies raised against ligand or antisense peptides as as defined above, or anti-anti-idiotype antibodies raised against ligand or antisense peptides can be prepared as mentioned above.

In this respect, according to another embodiment, the present invention relates to methods and processes for screening new anti-hepatitis drugs.

Life threatening inflammatory reactions can be induced by trauma and bacterial, viral and fungal infections. For example sepsis is such an important medical life-threatening syndrome characterized by chills, profuse sweating, fever, weakness or hypotension, leukopenia, intravascular coagulation, multiple organ failure and often death (Ulevitch et al., 1990). Lipopolysaccharides (LPS) from gram-negative bacteria are well known inducers of sepsis, but cell wall substances from gram-positive and fungal origin can cause this syndrome as well. Cytokines like TNF play an important role in the development of the disease (Beutler et al., 1985). Other LPS-like induced diseases include ARDS, acute pancreatitis, acute and chronic liver failure, intestinal and liver transplantation, inflammatory bowel disease, graft vs. host disease following bone marrow transplantation and tuberculosis.

The invention also relates to methods for screening drugs which can be used in the field of HBV infection and in the field of inflammatory reactions in patients.

More specifically, the invention relates to a method for identifying compounds interfering with the interaction between CD14 and HBV components comprising the steps of:
a) contacting the compound to be screened with a complementary set of molecules of claim 1, and,
b) detecting the binding affinity of said complementary set of molecules of claim 1 in the presence or absence of said compound to be tested.

The invention further relates to a host cell transformed with a nucleic acid sequence coding for muteins of CD14 or parts of CD14 which bind to HBV components, with said host expressing said insert polypeptides or peptides on its surface.

It should be understood that the host cell has been transformed with a recombinant vector as defined earlier.

The invention also relates to a method for identifying compounds interfering with the interaction between CD14 and HBV components comprising the steps of:
a) contacting the compound to be screened with a cellular host of claim 6, and,
b) detecting the formation of a complex between said compound and said CD14 molecule or a mutein or fragment thereof, or,
c) detecting the interference of said compound with the binding of HBV components with said CD14 molecule or a mutein or a fragment thereof.

The invention further relates to the above-mentioned method, wherein said detection step in b) or c) is a quantitative detection step to determine the affinity of binding said compound.

The invention also relates to the compounds identified by the above described methods. These compounds may be further modified for use in a delivery system. Said modifications may include the binding of the compounds to beads or to a targeting molecule such as an antibody, eventually by means of a spacer molecule. Also any other method for modification of molecules known in the art for purposes of delivery can be applied.

In a further embodiment the invention relates to a method for preventing, treating or alleviating inflammatory reactions in patients comprising the delivery of a compound according to any of claims 9 or 10.

The term "delivery of a compound" relates to methods of delivery or administration of such compound via oral, enteral or parenteral route such as for instance tablets, capsules, ampoules for injection or any other method known for purpose of delivery. The compound may as well be delivered or administered by means of controlled-release devices and methods.

The invention also relates to the use of a cellular host transformed with a nucleic acid sequence coding for CD14, muteins of CD14 or parts of CD14 which bind to HBV components for identifying compounds which interfere with the binding of HBV to its natural receptor.

Host cells expressing CD 14 have been described for instance by Golenbock et al. (1993), Jack et al. (1995), Kravchenko et al. (1996) and Devitt et al. (1998).

The invention also relates to the use of a molecule as defined in claim 1 for preventing, treating or alleviating HBV infection.

In the alternative the invention relates to the use of a molecule as defined in claim 1 for the manufacture of a medicament for treating, preventing or alleviating HBV infections.

The invention further relates to the use of a molecule as defined in claim 1 for the preparation of a screening assay for identifying useful compounds to prevent, treat or alleviate HBV infection.

The invention also relates to the use of a molecule as defined in claim 4 for preventing, treating or alleviating inflammatory reactions.

In the alternative the invention also relates to the use of HBV components in the manufacture of a medicament for treating, preventing or alleviating inflammatory reactions.

According to yet another embodiment, the invention relates to a host cell according to claim 6 for use as a model system for testing potential drugs which are able to exert at least one of the following functions:
a) modulate the binding or the affinity of binding of HBV components to said host cell,
b) modulate the expression of CD14 or its muteins or its fragments on the surface of said host cell,
c) modulate the activity of HBV components or the activity of CD14 or its muteins or fragments thereof, or
d) interfere with the replication of HBV in said host cell.

A preferred example of said model system is an in vitro assay to test possible drugs which can be used in the field of HBV infection or inflammation reactions.

The invention also relates to a mammalian non-human transgenic animal able of being infected by HBV and possibly suffering from hepatocellular damage caused by the consecutive or inducible expression of a nucleic acid encoding a CD14 mutein or a part of CD14 introduced into the genome of said transgenic animal, as a model system for testing potential drugs which are able to exert at least one of the following functions:
a) modulate the binding or the affinity of binding of HBV to a cell of said transgenic animal,
b) modulate the expression of CD14 or its muteins or its fragments on the surface of a cell of said transgenic animal,
c) modulate the activity of HBV components or the activity of CD14 or its muteins or fragments thereof, or
d) interfere with the replication of HBV in said transgenic animal.

A transgenic non-human mamalian animal can be prepared according to the protocol described in International Review of Cytology, Gordon JW, vol. 115, p. 171-222 (1989). Transgenic mice expressing CD14 have been described by Ferrero et al. (1993), Hetherington et al. (1998) and Tamura et al. (1999).

The invention also relates to a method for identifying potential HBV drugs comprising the use of a mammalian non-human transgenic animal wherein the animal CD14 gene is replaced by a human CD14 gene or wherein at least part of a human CD14 gene is introduced. Also the compounds identified by this method are part of the invention.

According to another embodiment, the invention relates to a method for the production of a composition comprising providing a compound identified by a any method as defined above and possibly mixing said identified compound with a pharmaceutically acceptable carrier.

Pharmaceutical compositions according to the present invention, and for use in accordance to the present invention, may comprise, in addition to the compound, a pharmaceutically acceptable excipient, carrier, buffer, stabiliser or other materials well known to those skilled in the art. Such materials should be non-toxic and should not interfere with the efficacy of the active ingredient. The precise nature of the carrier or other material may depend on the route of administration. Those of relevant skill in the art are well able to prepare suitable solutions.

The invention further relates to a method for preventing, treating or alleviating HBV infections comprising the delivery of a compound identified by the method of any of claims 7, 8 or 18.

The invention also relates to a model system for testing potential anti-HBV drugs comprising a mammalian non-human transgenic animal as defined in claim 18, said transgenic animal infected with HBV and showing symptoms related to HBV.

The invention, now being generally described, will be more readily understood by reference to the following examples, which are included merely for purposes of illustration of certain aspects and embodiments of the present invention and are not intended to limit the invention. All of the references mentioned herein are incorporated by reference.

### BRIEF DESCRIPTION OF FIGURES

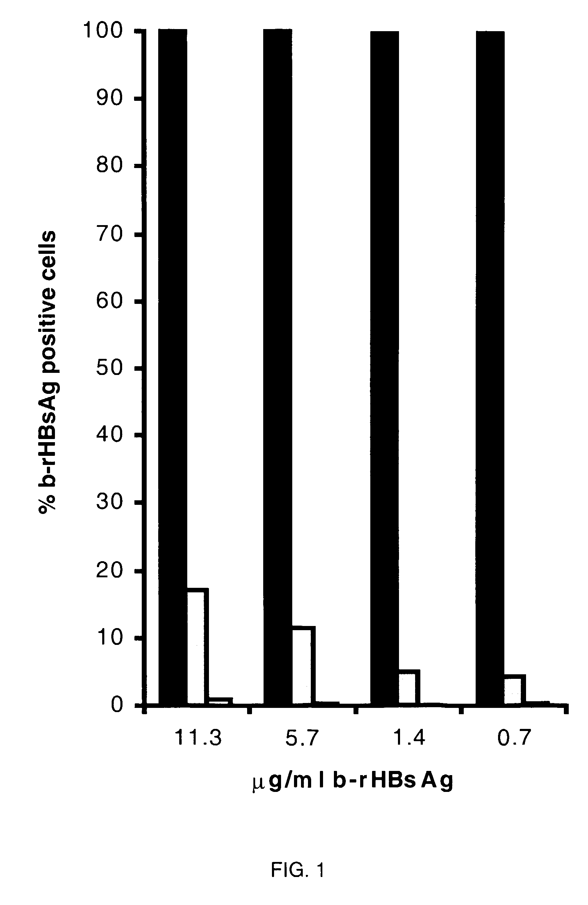

***Figure 1:*** Attachment of b-rHBsAg to CD14 positive PBMC
   Cells were incubated for 1 h with different concentrations of b-rHBsAg in 200 µl 2% HS-HBSS, washed twice with the same buffer and incubated with anti CD14-FITC (clone P9), anti CD19-FITC or anti CD3-FITC and Strep-PE. After two washes cells were analyzed. Percentage of b-rHBsAg positive CD14 (black bars), CD19 (grey bars) or CD3 (with bars) positive cells was determined.

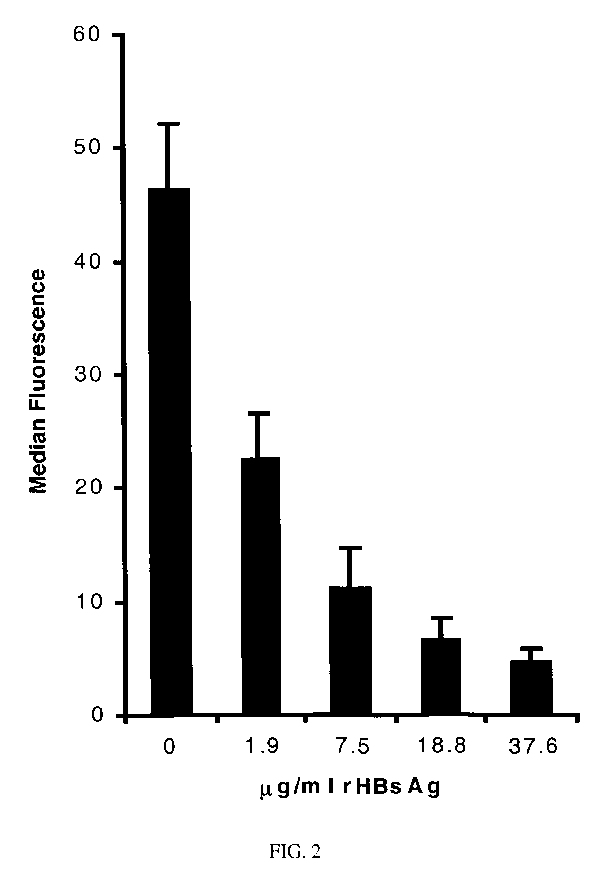

***Figure 2:*** Non-biotinylated rHBsAg competes with attachment of b-rHBsAg to monocytes.
   PBMC were incubated with different amounts of rHBsAg in 200 µl 2% HS-HBSS. After 1 h b-rHBsAg was added and the cells were incubated for another hour. After two washes cells were stained with Strep-PE, washed twice and analyzed. Median fluorescence was determined. The data shown represent the average of three seperate experiments. Error bars represent SD.

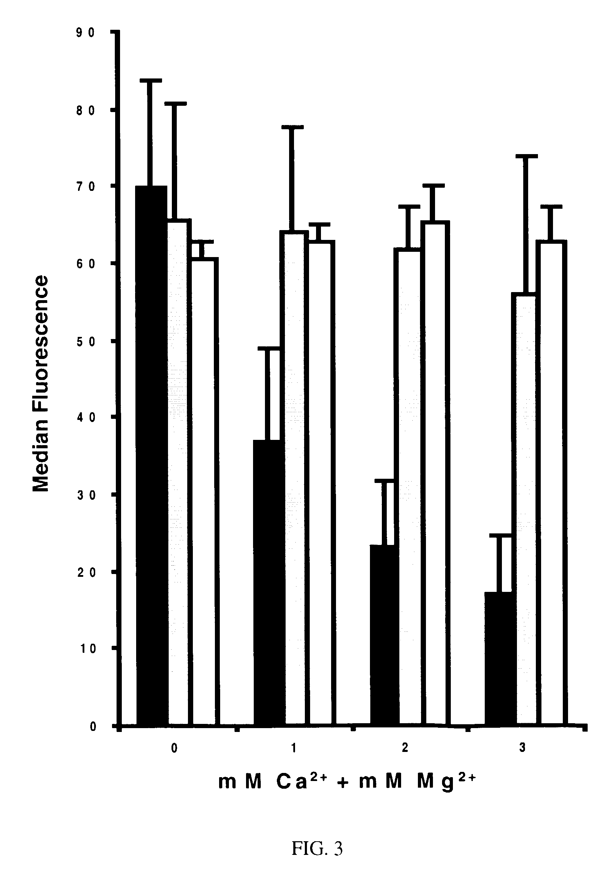

***Figure 3:*** Ca²⁺ and Mg²⁺ interfere with attachment of b-rHBsAg to monocytes.
   PBMC were incubated with b-rHBsAg in the presence of Ca²⁺ and Mg²⁺ (black bars) or Ca²⁺ and Mg²⁺ and 5 mM EDTA (grey bars) in 2% HS-50 mM Tris.HCI-150 mM NaCI, pH 7.4. After two washes with the same buffer cells were stained with Strep-PE, washed twice and analyzed. Median fluorescence was determined. The data shown represent the average of two seperate experiments. Error bars represent SD. Ca²⁺ and Mg²⁺ added after attachment of b-rHBsAg (white bars) does not reverse binding.

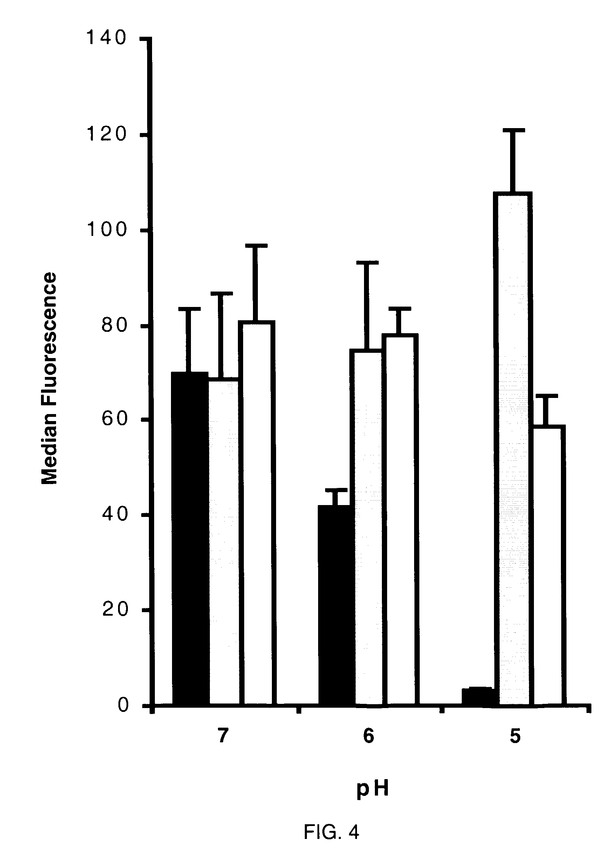

***Figure 4:*** Low pH prevents attachment of b-rHBsAg to monocytes.
   PBMC were incubated with b-rHBsAg in 2% HS-HBSS or 2% HS-10 mM citrate buffer-150 mM NaCI, pH 6 and 5 (black bars). After one wash step with the same buffer and one with 2% HS-HBSS, cells were stained with Strep-PE, washed twice and analyzed. Median fluorescence was determined. The data shown represent the average of two seperate experiments. Error bars represent SD. Pretreatment of PBMC (grey bars) or b-rHBsAg (white bars) with the same buffers does not prevent binding.

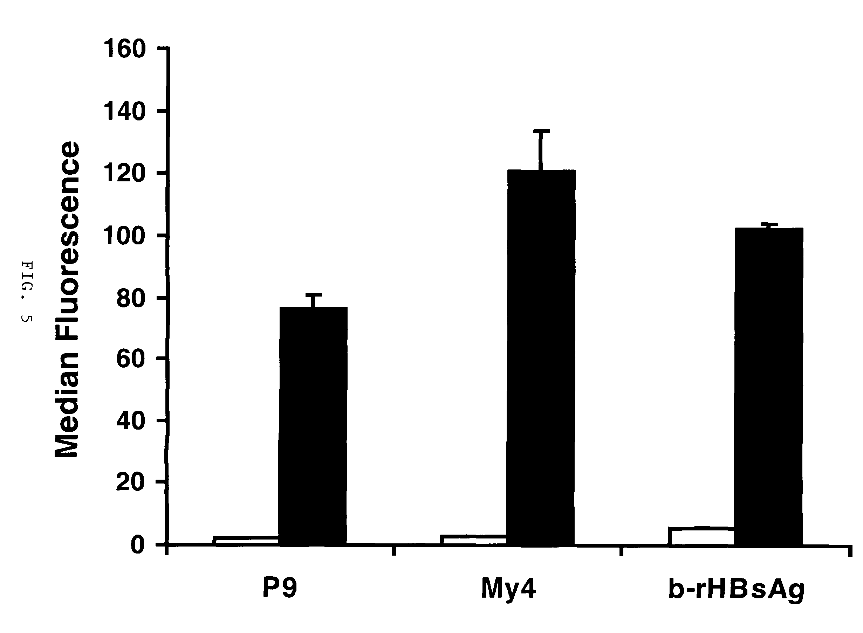

***Figure 5:*** 1,25-dihydroxyvitamineD3 differentiated THP-1 cells express CD14 and bind b-rHBsAg.
   Non-differentiated THP-1 cells (white bars) or differentiated cells (black bars) were incubated with anti CD14-FITC clone P9, clone MY4 or b-rHBsAg in 2% HS-HBSS. After two washes with the same buffer b-rHBsAg was detected with Strep-PE. Cells were washed twice and analyzed. Median fluorescence was determined. The data shown represent the average of two seperate experiments. Error bars represent SD.

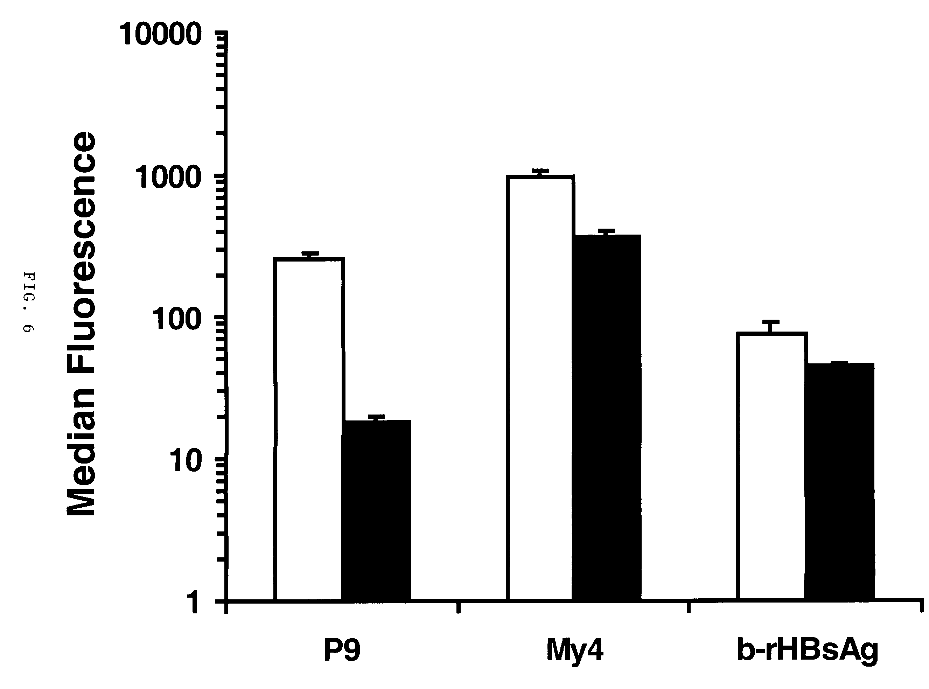

***Figure 6:*** b-rHBsAg attachment to PMA treated monocytes correlates with the expression level of My4 recognized CD14 molecules.
   Non-treated cells (white bars) and PMA treated cells (black bars) were stained with anti CD14-FITC clone P9, clone MY4 or b-rHBsAg in 2% HS-HBSS. After two washes with the same buffer b-rHBsAg was detected with Strep-PE. Cells were washed twice and analyzed. Median fluorescence was determined. The data shown represent the average of two seperate experiments. Error bars represent SD.

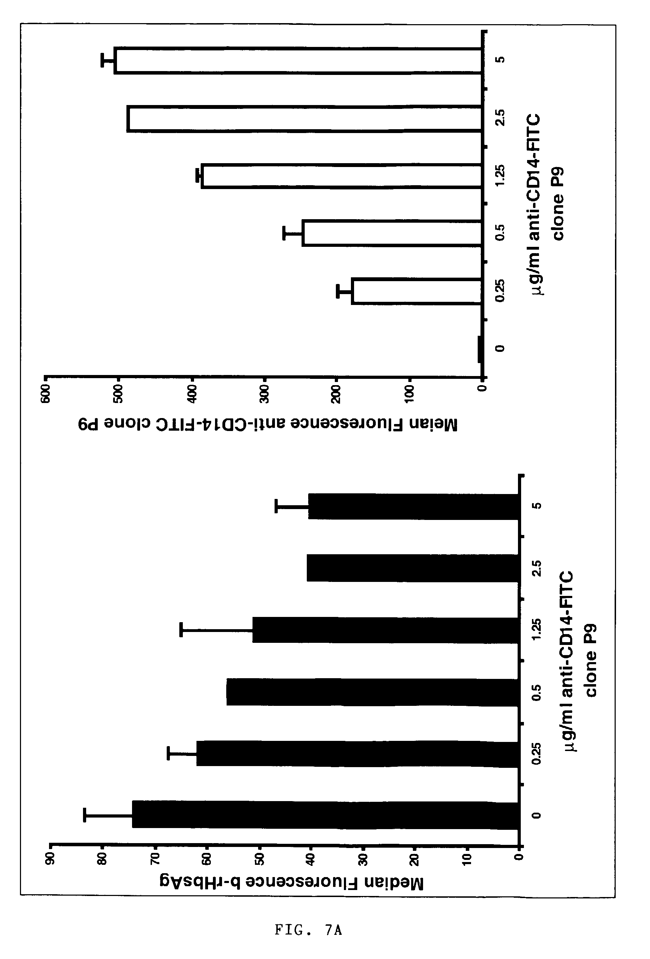

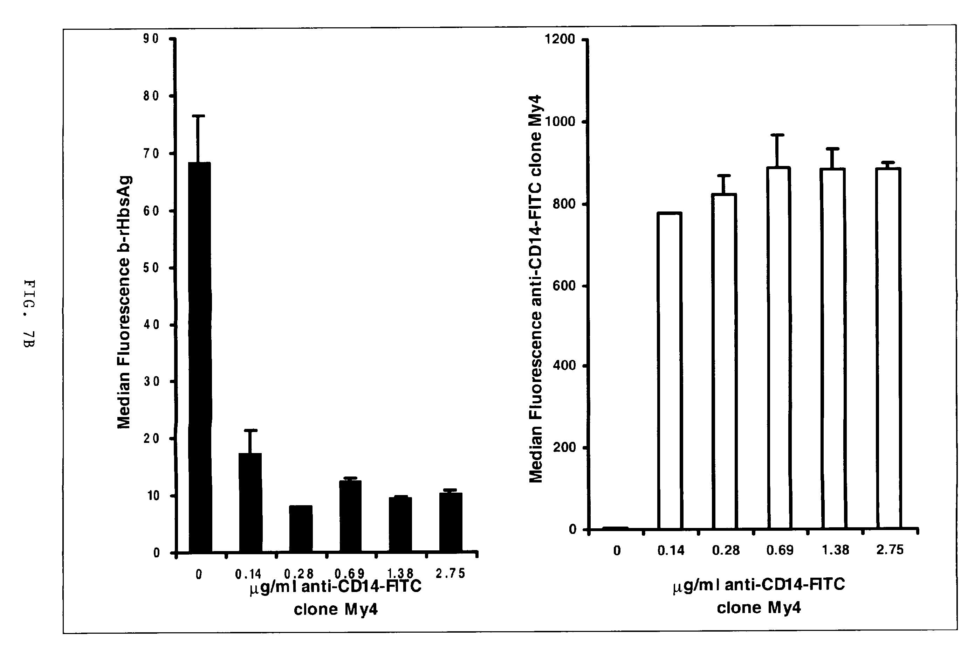

***Figure 7:*** CD14 specific monoclonal antibodies block attachment of b-rHBsAg to monocytes.
   PBMC were incubated with different amounts of anti CD14 clone P9 (7a) or My4 (7b) in 2% HS-HBSS. After two washes with the same buffer PBMC were incubate with b-rHBsAg, washed twice and stained with Strep-PE. Cells were washed twice and analyzed. Median fluorescence was determined. The data shown represent the average of two seperate experiments. Error bars represent SD. Left panel shows the effect of pre-incubation with anti CD14 mAbs on the attachment of b-rHBsAg; right panel shows the level of binding of these mAbs to monocytes.

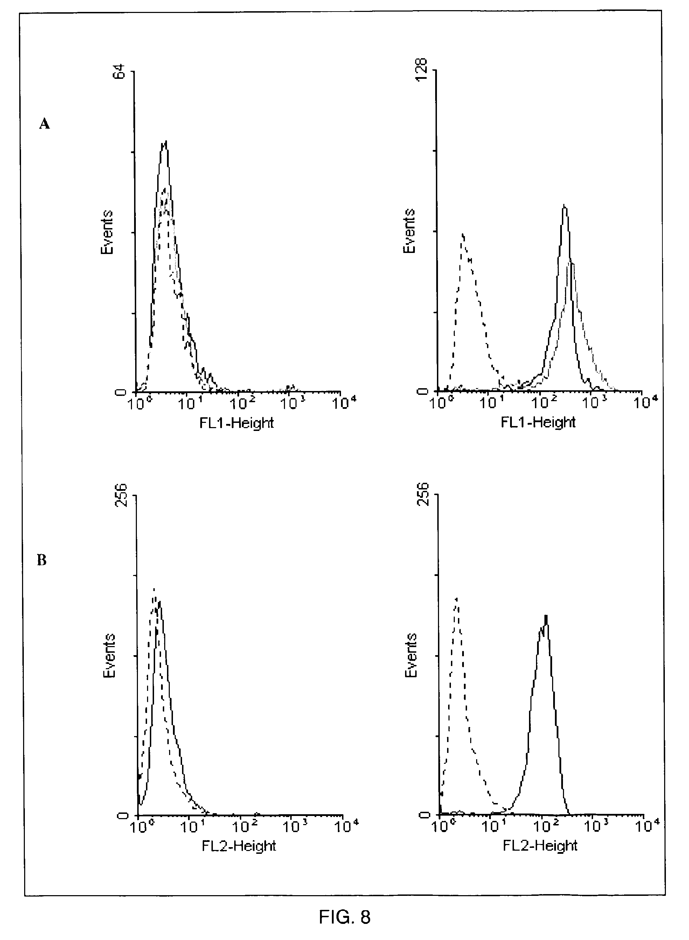

***Figure 8:*** b-rHBsAg binds to CHO cells which express CD14 from cDNA.
   CHO cells transfected with the empty plasmid (left panels) or cells expressing human CD14 (right panels) were incubated with anti CD14-FITC clone P9 (a, grey line), clone MY4 (a, full line) or b-rHBsAg (b, full line) in 2% HS-HBSS. After two washes with the same buffer b-rHBsAg was detected with Strep-PE. Cells were washed twice and analyzed. Dashed lines represent cells stained with isotypic controls or Strep-PE only.

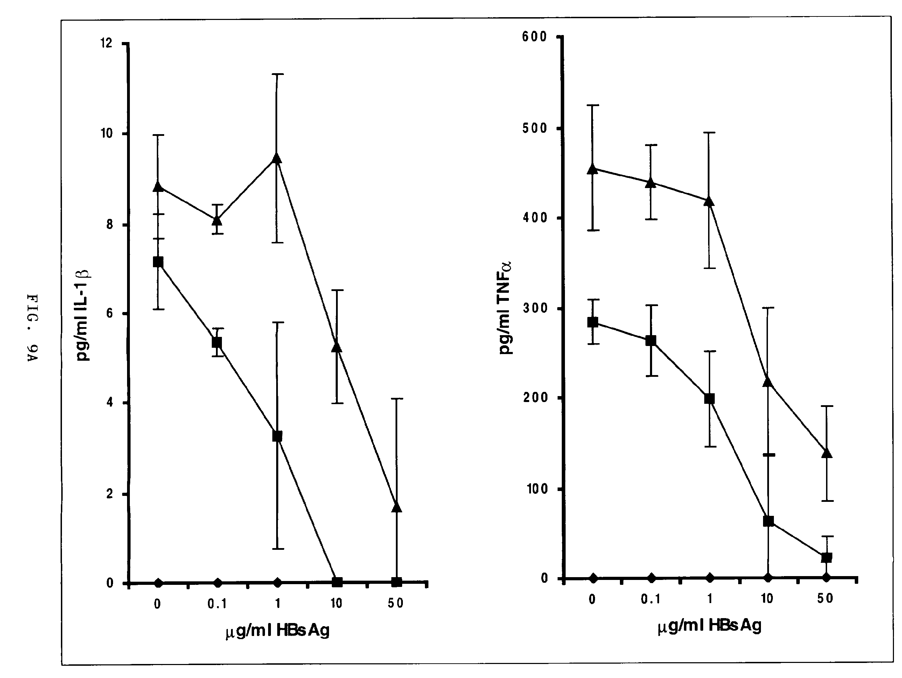

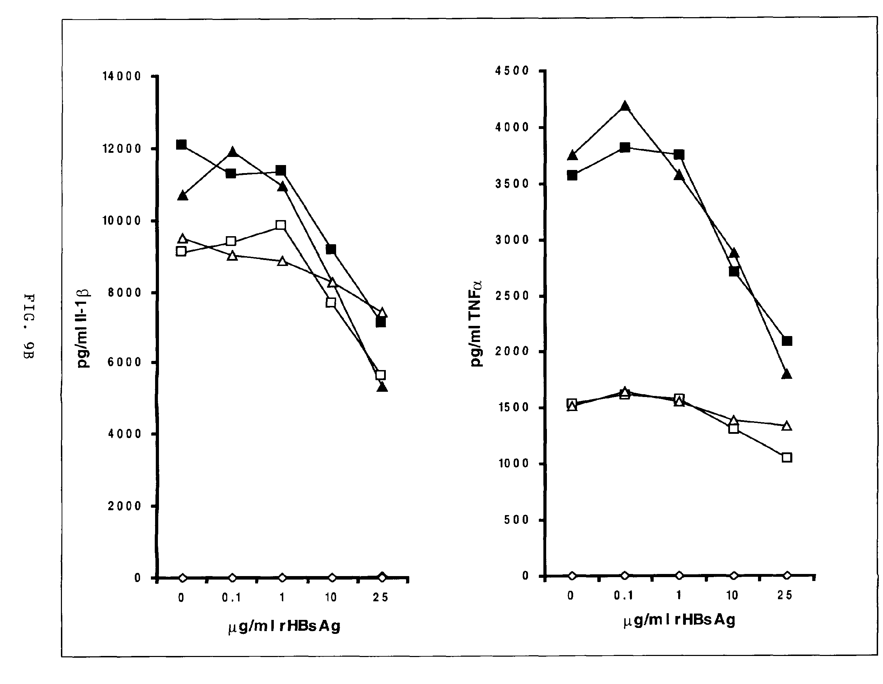

***Figure 9:*** rHBsAg reduces LPS induced II-1β and TNFα secretion.
   1,25-dihydroxyvitamineD3 differentiated THP-1 cells (a) or PBMC from donor 1 (black symbols) and donor 2 (white symbols) (b) were incubated with 0 (◆), 10 (■) or 50 (▲) ng/ml LPS and different concentrations of rHBsAg. Cell supernatant was collected after 24 h and cytokine concentrations determined.

### EXAMPLES

### Example 1 : Materials and methods

### Biotinylation of rHBsAg

rHBsAg (subtype adw₂) produced in *S. cerevisiae* (lot DVP23, 752 µg/ml in PBS, Smithkline Beecham Biologicals, Rixensart, Belgium) was biotinylated using an ECL protein biotinylation module (RPN 2202, Amersham Pharmacia Biotech). 300 µl rHBsAg was mixed with 270 µl H20, 30 µl 0.8 M bicarbonate buffer pH 8.6 and 15 µl biotinylation reagent. The mixture was incubated at RT for 1 h, after which 24 µl 1 M Tris was added. Biotinylated rHBsAg (b-rHBsAg) was purified by gel filtration on a Sephadex G25 column using PBS. 1 ml fractions were collected and b-rHBsAg peak fractions were determined by ELISA. Another rHBsAg lot used was DVP93.

### Antibodies

Mouse anti-human CD3-FITC (clone SK7), CD14-FITC (clone M(P9), CD19-FITC (clone 4G7), IgG1-FITC isotype control and Streptavidine-phycoerythrin (Strep-PE) were from Becton Dickinson. Mouse anti-human CD14-FITC (clone My4) and IgG2b-FITC isotype control were from Immunotech. Mouse anti-human CD18-FITC (clone 6.7) was from Pharmingen.

### ELISA

Maxisorp 96-well plates (Nunc, Roskilde, Denmark) were coated with B-rHBsAg in PBS. The wells were blocked with 0.1% BSA in PBS, followed by three washings (0.1% Triton X-100). Streptavidin-horse radish peroxidase (1/1000 in PBS-0.1% BSA) was added and the plates were incubated for 1 h at room temperature. After three washings 3.3',5,5'-tetramethylbenzidine (Sigma) was added. After 30 minutes the reaction was stopped with 0.1 N H₂SO₄.

### Cells

Human PBMC were isolated from buffy coats using Ficoll-Hypaque (density=1.077 g/ml, Nycomed Pharma, Oslo, Norway) centrifugation. Cells were stored in liquid nitrogen. Phorbol-12-myristate-13-acetate treatment (25 ng/ml) (PMA, Sigma) was performed for 4 h in cRPMI (RPMI 1640-10% FCS-2mM L-glutamine-1 mM Na-pyruvate-50 U/ml penicilline-50 µg/ml streptomycine-20 µM β-mercaptoethanol). THP-1 cells were grown in complete cRPMI. To induce differentiation 100 nM 1,25-dihydroxyvitamin D3 (1,25-VitD3, Calbiochem) was added for 48 h. CHO cells expressing human CD14 and CHO cells transfected with the vector only (Jack et al., 1995) were grown in MEM alpha without nucleosides and ribonucleosides (Gibco-BRL) supplemented with 10% FCS, 2mM L-glutamine-50 U/ml penicilline-50 µg/ml streptomycine-100 nM (plasmid transfected CHO cells) or 500 nM methotrexate (CD14 expressing CHO cells). Cultured cells were detached mechanically or by using non enzymatic cell dissociation buffer (Sigma), washed twice with 2% HS-HBSS and stained as described below.

### Staining of cells

PBMC were thawed and washed twice with 2% HS-HBSS. 10⁶ cells were incubated with b-rHBsAg in 200 µl 2% HS-HBSS for 1 h on ice. After 2 washes with the same solution, cells were incubated with Strep-PE and/or FITC labelled antibodies in 2% HS-HBSS for 1 h on ice. After 2 washes cells were resuspended in 1 ml 2% HS-HBSS or PBS, containing propidium iodide (PI) and analysed on a FACScan flow cytometer (Becton Dickinson). Dead cells which incorporated PI were gated out of analysis. At least 5000 cells were counted per analysis. Fluorescence (530 nm for FITC and 580 nm for PE) was measured. Median fluorescence was determined in each case. The signals were acquired in a logarithmic mode for FI1 (FITC) and FI2 (PE). Treshhold levels were set according to negative (Strep-PE only) and isotypic controls. Gates for monocytes were set to the position in the SSC and the FSC.

### LPS treatment of THP-1 and PBMC.

5.10⁵ THP-1 cells were treated for 24 h with 100 nM 1,25-dihydroxyvitamin D3 (1,25-VitD3, Calbiochem). After washing, the cells were incubated in cRPMI with or without 10 or 50 ng/ml LPS (E. coli 0111:B4, Sigma). 0, 0.1, 1, 10 or 50 µg/ml rHBsAg was added. 10⁶ PBMC were incubated in cRPMI with or without 10 or 50 ng/ml LPS. 0, 0.1, 1, 10 or 25 µg/ml rHBsAg was added. Cell supernantants were collected after 24 h and tested for the presence of II-1β and TNFα.

### Cytokine determinations.

The concentration of IL-1β and TNFα in cell supernatant was determined using commercially availble kits (Bioscource) according to the manufactors instructions.

### Example 2 : Characterization of b-rHBsAg binding to PBMC.

rHBsAg was biotinylated and purified as described. Two b-rHBsAg peak fractions were determined by ELISA. b-rHBsAg was still recognized in a HBsAg specific radioimmunoassay (AUSRIA Abott Laboratories, Chicago Illinois) and by ELISA using monoclonal antibodies specific for the 'a' and 'd' determinant. This ELISA was also used to determine the concentration of b-rHBsAg in the peak fractions (data not shown). These results show that biotinylation does not alter the antigenic structure of the particles.

PBMC were incubated with different concentrations of b-rHBsAg after which the cells were incubated with mAbs specific for monocytes, T or B-cells. b-rHBsAg was detected with Strep-PE. At the lowest b-rHBsAg concentration almost only CD14+ cells were b-rHBsAg positive. At higher concentrations a fraction of CD19+ cells became positive for b-rHBsAg as well. Binding of b-rHBsAg to CD3+ cells was never detected (Fig. 1). Based on these results it was decided to use approximately 1-2 µg/ml b-rHBsAg in all experiments. The optimal serum concentration for binding was subsequently determined to be 2-3%. Human serum could be replaced by foetal calf serum and BSA (data not shown), ruling out the involvement of a specific serum protein.

To demonstrate the specificty of the observed interaction, PBMC were incubated with different amounts of non-biotinylated rHBsAg. After 1 h b-rHBsAg was added to this mixture. As shown in Fig. 2, unlabelled rHBsAg blocked the binding of b-rHBsAg. Inhibition was also observed when non-biotinylated rHBsAg from lot DVP93 was used (data not shown).

The interaction of HBV proteins with cells or putative cellular receptors has often been claimed to be Ca²⁺ dependent or independent. (Hertogs et al., 1993; Neurath et al., 1990; Komai and Peeples, 1990; Mehdi et al., 1996). Contrary to these reports, the addition of increasing amounts of Ca²⁺ and Mg²⁺ caused reduced binding of b-rHBsAg. The addition of 5 mM EDTA to the mixture restored attachment. The addition of Ca²⁺ and Mg²⁺ after binding had no effect (Fig. 3). Reduced binding was also observed when only Ca²⁺ or only Mg²⁺ was added (results not shown). These experiments were all performed in 2% HS-50 mM Tris.HCI-150 mM NaCI instead of HBSS to prevent acidification when adding 5 mM EDTA.

Conflicting results about the influence of low pH on virus infection or HBsAg attachment have been reported. (Komai and Peeples, 1990; Mehdi et al., 1996; Lu et al., 1996; Hagelstein et al., 1997). Therefor the effect of pH on particle binding was measured at pH 7, 6 and 5. As shown in Fig.4 b-rHBsAg binding was strongly reduced when the pH was lowered. Pre-incubation of the cells or b-rHBsAg at the same pH and for the same time did not have any effect.

### Example 3 : Effect of CD14 expression levels on b-rHBsAg binding.

In initial experiments monocytes were stained with anti CD14-FITC clone P9 after incubation with b-rHBsAg. A slightly decreased binding of this mAb was noticed compared to the attachment to cells which were not pre-incubated with b-rHBsAg (data not shown). This observation raised the possibility that rHBsAg would bind (close) to CD14 molecules. A first approach to obtain evidence for this hypothesis was to check the binding of b-rHBsAg to a pre-monocytic cell line which does not express CD14. CD14 expression was detected using two different specific mAbs, clone P9 and My4. Both antibodies were used because they recognize different forms of CD14, which may differ in expression levels on monocytes and monocytic cell lines (Pedron et al., 1995). As expected, undifferentiated THP-1 cells showed no detectable expression of CD14 and did not bind b-rHBsAg. 1,25-VitD3 differentiated THP-1 cells expressed CD14 molecules which were recognized by both antibodies. These differentiated cells did bind b-rHBsAg (Fig.5).

As a second approach the effect of PMA treatment on b-rHBsAg binding to PBMC was examined. An established feature of CD14 is that monocytes shed it rapidly when treated with PMA (Bazil and Strominger, 1991). Again anti CD14 antibodies, clone P9 and My4, were used to detect changes in CD14 levels. Similar to previous reports (Pedron et al., 1995) PMA treatment removed CD14 molecules recognized by clone P9 almost completely, while CD14 molecules recognized by clone My4, although reduced, showed still a high expression level (Fig. 6). b-rHBsAg attachment to the PMA treated monocytes was only slightly reduced. Taken together these results suggest that b-rHBsAg could bind to My4 recognized CD14 molecules.

### Example 4 : Effect of CD14 specific antibodies on b-rHBsAg binding.

The ability of the anti CD14 antibodies to block the attachment of b-rHBsAg to monocytes was examined. PBMC were incubated with increasing amounts of anti CD14 clone My4 or P9. After washing, the cells were incubated with b-rHBsAg. As shown in Fig. 7a partial inhibition of b-rHBsAg attachment was obtained when cells were pre-incubated with increased concentrations of antibody P9. Even at the lowest concentration of antibody My4 almost complete inhibition of b-rHBsAg binding was obtained (Fig. 7b). A mAb directed against CD18, another LPS binding molecule, had no effect on b-rHBsAg attachment (data not shown).

### Example 5 : Expression of CD14 in a non-monocytic cell line.

Previous results strongly suggested that b-rHBsAg bind to CD14. The ability of CD14 to bind b-rHBsAg when expressed in a non-monocytic cell line was examined. CHO cells expressing human CD14 or cells transfected with the empty plasmid were kindly provided by Dr. Stelter (Jack et al., 1995). As shown in Fig. 8 both CD14 specific mAbs did recognize the CD14 transfected cells and not the control cells. Attachment of b-rHBsAg was also only observed to CHO cells which expressed CD14.

### Example 6: Effect of HBsAg on LPS induced cytokine production.

THP-1 were incubated for 24 h with 100 nM 1,25-VitD3, washed and incubated with or without LPS in the presence of different HBsAg concentrations. Cell supernatant was collected after 24 h of incubation and tested for the presence of II-1β and TNFα. As shown in Fig. 9a HBsAg alone did not induce any cytokine production while LPS induced the secretion of the 2 cytokines. High levels of TNFα were produced while lower concentrations of II-1β were detected. Highest cytokine levels were obtained with 50 ng/ml LPS. Cytokine concentrations were dose-dependently decreased in the presence of HBsAg.

The effect of HBsAg on LPS induced cytokine production was also studied with PBMC from two different donors. As shown in Fig 9b PBMC of donor 1 produced higher amounts of TNFα compared to the PBMC from donor 2. Nevertheless, dose-dependent reduction of TNF levels in the presence of HBsAg was more pronounced with PBMC form donor 1. Similar results were obtained for II-1β.

### REFERENCES

Adkins, J. and Wagstaff, A. (1998). Recombinant Hepatitis B vaccine. A review of its immunogenecity and protective efficacy against hepatitis B. *Biodrugs* **10,** 137-158.

Bazil, V., and Strominger, J. L. (1991). Shedding as a mechanism of down-modulation of CD14 on stimulated human monocytes. *J Immunol* **147**(5), 1567-74.

Beutler, B., Milsark I., and Cerami, A. (1985). Passive immunization against cachectin/tumor necrosis factor protects mice from lethal effect of endotoxin. *Science* **229(4716),** 869-71

Blum, H., Stowring, L., Figus, A., Montgomery, C., Haase, A., and Vyas, G. (1983). Detection of HBV DNA in hepatocytes, bile duct epithelium and vascular elements in in situ hybridization. *Proc Natl Acad Sci* **80**, 6685-88.

Blumberg, B. (1997). Hepatitis B virus, the vaccine, and the control pf primary cancer of the liver. *Proc. Natl. Acad. Sci. USA* **94,** 7121-5.

Bosco, M. C., Espinoza-Delgado, I., Rowe, T. K., Malabarba, M. G., Longo, D. L., and Varesio, L. (1997). Functional role for the myeloid differentiation antigen CD14 in the activation of human monocytes by IL-2. J *Immunol* **159**(6), 2922-31.

Budkowska, A., Bedossa, P., Groh, F., Louise, A., and Pillot, J. (1995). Fibronectin of human liver sinusoids binds hepatitis B virus: identification by an anti-idiotypic antibody bearing the internal image of the pre-S2 domain. *J Virol* **69,** 840-48.

Bufler, P., Stiegler, G., Schuchmann, M., Hess, S., Krüger, S., Stelter, F., Eckerskorn, C., Schütt, C, and Engelmann, H. (1995). Soluble lipopolysaccharide receptor (CD14) is released via two different mechanisms from human monocytes and CD14 transfectants. *Eur. J. Immunol.***25,** 604-610.

Cavanaugh, V., Guidotti, L., and Chisari (1998). Inhibition of hepatitis B virus replication during adenovirus and cytomegalovirus infections in transgenic mice. *J Virol* **72**, 2630-7.

Dejean, A., Lugassy, C., Zafrani, S., Tiollais, P., and Brechot, C. (1984). Detection of hepatitis B virus DNA in pancreas , kidney and skin of two human carriers of the virus. *J Gen Virol* **65**, 651-55.

Devitt, A., Moffatt, O. D., Raykundalia, C., Capra, J. D., Simmons, D. L., and Gregory, C. D. (1998). Human CD14 mediates recognition and phagocytosis of apoptotic cells. *Nature* **392**, 505-509.

Ellis, R.W. (1993) "Hepatitis B vaccines in Clinical practice" edited by Marcel Dekker, Inc, New York, USA.

Fadok, V. A. (1998). Macrophages that have ingested apoptotic cells in vitro inhibit proinflammatory cytokine production through autocrine/paracrine mechanisms involving TGF-□ PGE2 and PAF. *J Clin Invest* **101**, 890-98.

Fearns, C., Kravchenko, V., Ulevitch, R., and Loskutoff, D. (1995). Murine CD14 gene expression in vivo: extramyeloid synthesis and regulation by lipoploysaccharide. *J. Exp. Med.* **181**, 857.

Ferrari, C., Penna, A., Bertoletti, A., Valli, A., Antoni, A. D., Giuberti, T., Cavalli, A., Petit, M. A., and Fiaccadori, F. (1990). Cellular immune response to hepatitis B virus-encoded antigens in acute and chronic hepatitis B virus infection. *J Immunol* **145**(10), 3442-49.

Ferrero, E., Hsieh, C., Francke, U., and Goyert, S. (1990). CD14 ids a member of the family of leucine-rich proteins and is encoded by a gene syntenic with multiple receptor genes. *J Immunol* **145,** 331-36.

Ferrero, E., Jiao, D., Tsuberi, B., Tesio, L., Rong, G., Haziot, A., Goyert, S. (1993) Transgenic mice expressing human CD14 are hypersensitive to lipopolysaccharide. *Proc. Natl. Acad. Sci. U S A* **90**, 2380-4

Franco, A., Paroli, M., Tresta, U., Benvenuto, R., Peschle, C., Balsano, F., and Barnaba, V. (1992). Transferrin receptor mediates uptake and presentation of hepatitis B envelope antigen by T lymphocytes. *J Exp Med* **175,** 1095-105.

Gerlich, W. and Bruss, V. F (1993). Functions of hepatitis B virus proteins and molecular targets for protective immunity. In "Hepatitis B vaccines in Clinical practice" edited by Ellis, R.W. Marcel Dekker, Inc, New York, USA.

Gerlich, W. H., Lu, X., and Heermann, K. H. (1993b). Studies on the attachment and penetration of hepatitis B virus. *J Hepatol* **17**(3), S10-14.

Golenbock, D.,, Liu, Y., Millham, F., Freeman, M., and Zoeller, R. (1993). Surface expression of human CD14 in Chinese hamster ovary fibroblasts imparts macrophage-like responsiveness to bacterial endotoxin. *J. Biol. Chem.* **268,** 22055-9

Goyert, S., Ferrero, E., Seremitis, S., Winchester, R., Silver, J. and Mattison, A., (1986). Biochemistry and expression of myelomonocytic antigens. *J. Immunol.,* **137,** 3909.

Hagelstein, J., Fathinejad, F., Stremmel, W., and Galle, P. R. (1997). pH-independent uptake of hepatitis B virus in primary human hepatocytes. *Virology* **229**, 292-94.

Harrison, T. J. (1990). Hepatitis B virus DNA in peripheral blood leukocytes: a brief review. *Journal of Medical Virology* **31**(1), 33-35.

Haziot, A., Chen, E., Ferrero, E., Low, M. G., Silber, R., and Goyert, S. M. (1988). The monocyte differentiation antigen, CD14, is anchored to the cell membrane by a phosphatidylinositol likage. *J Immunol* **141,** 547-52.

Heidenreich, S., Schmidt, M., August, C., Cullen, P., Rademaekers, A., and Pauels, H. G. (1997). Regulation of human monocyte apoptosis by the CD14 molecule. *J Immunol* **159**(7), 178-88.

Hertogs, K., Leenders, W. P. J., Depla, E., De Bruin, W. C. C., Meheus, L., Raymackers, J., Moshage, H., and Yap, S. H. (1993). Endonexin II, present on human liver plasma membranes, is a specific binding protein of small hepatitis B virus (HBV) envelope protein. *Virology* **197,** 549-57.

Hetherington, C. J., Kingsley, P. D., Crocicchio, F., Zhang, P., Rabin, M. S., Palis, J., and Zhang, D. E. (1999). Characterization of human endotoxin lipopolysaccharide receptor CD14 expression in transgenic mice. *J Immunol* **162**, 503-9.

Heumann, D., Barras, C., Severin, A., Glauser, M. P., and Tomasz, A. (1994). Gram positive cell walls stimulate synthesis of tumor necrosis factor alpha and interleukin-6 by human monocytes. *Infect Immun* **62**, 2715-21.

Imai, M., Yanase, Y., Nojiri, T., Miyakawa, Y., and Mayumi, M. (1979). A receptor for polymerized human and chimpanzee albumins on hepatitis B virus particles co-occurring with HBeAg. *Gastroenterology* **76**, 242-47.

Jabara, H. H., and Vercelli, D. (1994). Engagement of CD14 on monocytes inhibits the synthesis of human Igs, including IgE. *J Immunol* **153**(3), 972-78.

Jack, R. S., Grunwald, U., Stelter, F., Workalemahu, G., and Schutt, C. (1995). Both membrane-bound and soluble forms of CD14 bind to gram-negative bacteria. *European Journal of Immunology* **25**(5), 1436-1441.

Komai, K., and Peeples, M. E. (1990). Physiology and function of the vero cell receptor for the hepatitis B virus small S protein. *Virology* **177**, 332-38.

Korba, B. E., Cote, P. J., Wells, F. V., Baldwin, B., Popper, H., Purcell, R. H., Tennant, B. C., and Gerin, J. L. (1989). Natural history of woodchuck hepatitis virus infections during the course of experimental viral infection: Molecular virologic features of the liver and lymphoid tissues. *J Virol* **63,** 1360-70.

Kravchenko, V., Steinemann, S., Kline, L., Feng, L., and Ulevitch, R. (1996) Endotoxin tolerance is induced in Chinese hamster ovary cell lines expressing human CD14.*Shock* **5,**194-201.

Kusunoki, T., Hailman, E., Juan, T. S., Lichenstein, H. S., and Wright, S. D. (1995). Molecules from Staphylococcus aureus that bind CD14 and stimulate innate immune responses. *J Exp Med* **182,** 1673-82.

Labeta, M. O., Landmann, R., Obrecht, J. P., and Obrist, R. (1991). Human B cells express membrane-bound and soluble forms of the CD14 myeloid antigen. *Mol Immunol* **28**(1-2), 115-22.

Lu, X., Block, T. M., and Gerlich, W. H. (1996). Protease-induced infectivity of hepatitis B virus for a human hepatoblastoma cell line. *J Virol* **70**(4), 2277-85.

Lue, K. H., Lauener, R. P., Winchester, R. J., Geha, R. S., and Vercelli, D. (1991). Engagement of CD14 on human monocytes terminates T cell proliferation by delivering a negative signal to T cells. *J Immunol* **147,** 1134.

Malmqvist, M. (1999). BIACORE: an affinity biosensor system for characterization of biomolecular interactions.

Mason, A., Wick, M., White, H., and Perrillo, R. (1993). Hepatitis B virus replication in diverse cell types during chronic hepatitis B virus infection. *Hepatology* **18**(4), 781-89.

Matsuura, K., Setoguchi, M., Nasu, N., Higuchi, Y., Yoshida, S., Akizuki, S., and Yamamoto, S. (1989). Nucleotide and amino acid sequences of the mouse CD14 gene. *Nucleic Acids Res.* **17,** 2132.

Mehdi, M., Kaplan, M. J., Anlar, F. Y., Yang, X., Bayer, R., Sutherland, K., and Peeples, M. E. (1994). Hepatitis B virus surface antigen binds to apolipoprotein H. *J Virol* **68**(4), 2415-24.

Mehdi, H., Yang, X., and Peeples, M. E. (1996). An altered form of apolipoprotein H binds hepatitis B virus surface antigen most efficiently. *Virology* **217**, 58-66.

Moshage, H. (1997). Cytokines and the hepatic acute phase response. J *Pathol* **181,** 257.

Muller, C., and Zielinski, C. C. (1990). Impaired lipopolysaccharide-inducible tumor necrosis factor production in vitro by peripheral blood monocytes of patients with viral hepatitis. *Hepatology* **12**(5), 1118-24.

Muller, C., and Zielinski, C. C. (1992). Interleukin-6 production by peripheral blood monocytes in patients with chronic liver disease and acute viral hepatitis. *J Hepatol* **15**(3), 372-77.

Nagaraju, K., Naik, S. R., and Naik, S. (1998). Chronic hepatitis B virus carriers have low lymphoproliferative responses to HBsAg and reduced interleukin-2 synthesis. *Indian J Gastroenterol* **17**(3), 83-86.

Neurath, A. R., Strick, N., Sproul, P., Ralph, H. E., and Valinsky, J. (1990). Detection of receptors for hepatitis B virus on cells of extrahepatic origin. *Virology* **176**, 448-57.

Neurath, A. R., Strick, N., and Sproul, P. (1992). Search for hepatitis B virus cell receptors reveals binding sites for interleukin 6 on the virus envelope protein. *J Exp Med* **175,** 461-69.

Newman, S. L., Chaturvedi, S., and Klein, B. (1995). The W-1 antigen of blastomyces dermatitidis yeasts mediates binding to human macrophage CD11b/DC18 (CR3) and CD14. *J Immunol* **154,** 753-61.

Papaevangelou, G. (1998). Current combined vaccines with hepatits B. *Vaccine* **16suppl,** 69-72.

Pedron, T., Girard, R., and Chaby, R. (1995). Variation of LPS-binding capacity, epitope expression, and shedding of membrane-bound CD14 during differentiation of human monocytes. *J Immunol* **155,** 1460-71.

Petit, M. A., Capel, F., Dubanchet, S., and Mabit, H. (1992). PreS1 - specific binding proteins as potential receptors for hepatitis B virus in human hepatocytes. *Virology* **187,** 211-22.

Pontisso, P., Morsica, G., Ruvoletto, M. G., Zambello, R., Colletta, C., Chemello, L., and Alberti, A. (1991). Hepatitis B virus binds to peripheral blood mononuclear cells via the pre S1 protein. *J Hep* **12,** 203-6.

Pontisso, P., Ruoletto, M., Tiribelli, C., Gerlich, W., Ruol, A., and Alberti, A. (1992). The preS1 domain of hepatitis B virus and IgA cross-react in their binding to the hepatocyte surface. *J Gen Virol* **73,** 2041-45.

Setoguchi, M., Nasu, N., Yoshida, S., Higuchi, Y. Akizuki, S., and Yamamoto, S. (1989). Mouse and human CD14 (myeolid cell-specific leucine-rich glycoprotein) primary structure deduced from cDNA clones. *Biochim. Biophys. Acta* **1008,** 213-22.

Soell, M., Lett, E., Hoveck, F., Scholler, M., Wachsmann, D., and Klein, J. (1995). Activation of human monocytes by streptococcal rhamnose glucose polymers is mediated by CD14 antigen and mannan binding protein inhibits TNF-alpha release. *J Immunol* **154,** 852-60.

Stelter, F., Pfister, M., Bernheiden, M., Jack, R., Bufler, P., Engelmann, H., and Schutt, C. (1996). The myeloid differentiation antigen CD14 is N- and O-glycosylated. Contribution of N-linked glycosylation to different soluble CD14 isoforms. *Eur. J. Biochem.* **236,** 457-64.

Su, G. L., Dorko, K., Strom, S. C., Nussler, A. K., and Wang, S. C. (1999). CD14 expression and production by human hepatocytes. *J Hepatol* **31,** 435-42.

Tamura, Y., Higuchi, Y., Kataoka, M., Akizuki, S., Matsuura, K., and Yamamoto, S. (1999). CD14 transgenic mice expressing membrane and soluble forms: comparisons of levels of cytokines and lethalities in response to lipopolysaccharide between transgenic and non-transgenic mice. *Int. Immunol.* **11**,333-9

Treichel, U., Meyer zum Buschenfelde, K. H., Stockert, R. J., Poralla, T., and Gerken, G. (1994). The asialoglycoprotein receptor mediates hepatic binding and uptake of natural hepatitis B virus particles derived from viraemic carriers. *J Gen Virol* **75,** 3021-29.

Trippler, M., Meyer zum Buschenfelde, K. H., and Gerken, G. (1999). HBV viral load within subpopulations of peripheral blood mononuclear cells in HBV infection using limiting dilution PCR. *J Virol Meth* **78,** 129-47.

Ulevitch R., Mathison J., Schumann R., and Tobias P. (1990). .A new model of macrophage stimulation by bacterial lipopolysaccharide. *J Trauma* **30(12 Suppl),** S189-92.

Vingerhoets, J., Michielsen, P., Vanham, G., Bosmans, E., Paulij, W., Ramon, A., Pelckmans, P., Kestens, L., and Leroux-Roels, G. (1998). HBV-specific lymphoproliferative and cytokine responses in patients with chronic hepatitis B. *J Hepatol* **28**(1), 8-16.

Voll, R. E. (1997). Immunosuppressive effects of apoptotic cells. *Nature* **390,** 350-351.

Wright, S. D., Ramos, R. A., Tobias, P. S., Ulevitch, R. J., and Mathison, J. C. (1990). CD14, a receptor for complexes of lipopolysaccharide (LPS) and LPS binding protein. *Science* **249,** 1431.

Yoffe, B., Burns, D. K., Bhatt, H. S., and Combes, B. (1990). Extrahepatic hepatitis B virus DNA sequences in patients with acute hepatitis B infection. *Hepatology* **12**, 187-92.

Zeldis, J. B., Mugishima, H., Steinberg, H. N., Nir, E., and Gale, R. P. (1986). In vitro hepatitis B virus infection of human bone marrow cells. *Journal of Clinical Investigation* **78**(2), 411-417.

## Claims

1. Method for determining the binding between CD14 and Hepatitis B virus components comprising the use of:
a) CD14, or a mutein, or a fragment of CD14 which is able to bind to HBV antigens or particles,
b) a ligand of the molecules mentioned in a),
c) an antibody raised against the molecules mentioned in a),
d) an antibody raised against the ligand mentioned in b),
e) an anti-idiotype antibody raised against the antibody mentioned in c),
f) an anti-idiotype antibody raised against the antibody mentioned in d)
g) HBV components, or,
h) antibodies raised against the molecules or particles mentioned in g).

2. Composition for preventing, treating or alleviating HBV infections comprising as an active substance at least one of the following :
a) CD14 or a mutein or fragment thereof which binds to HBV components,
b) a ligand or antisense peptide of the molecules mentioned in a),
c) an antibody raised against the molecules mentioned in a),
d) an antibody raised against the ligand or antisense peptide mentioned in b),
e) an anti-idiotype antibody raised against the antibody mentioned in c),
f) an anti-idiotype antibody raised against the antibody mentioned in d),
g) fragments of HBV protein(s) which bind to CD14, and,
h) antibodies specifically recognizing the fragments mentioned in g).

3. Vaccine composition for preventing, treating or alleviating HBV infections comprising as an active substance at least one of the following :
a) receptor ligands or antisense peptides as defined in claim 1 or antibodies directed against these ligands or antisense peptides,
b) anti-idiotype antibodies raised against the receptor ligands or antisense peptides as defined in claim 1,
c) muteins or fragments of CD14 as defined in claim 1 or antibodies directed against these muteins or fragments,
d) immunogenic fragments of HBV antigens which interact with CD14 fragments comprising epitopes of antigens of HBV which bind to CD14, or,
e) antibodies specifically recognizing the fragments or d).

4. Composition for treating, preventing or alleviating inflammatory reactions in patients comprising the use of HBV components.

5. Method for identifying compounds interfering with the interaction between CD14 and HBV components comprising the steps of:
a) contacting the compound to be screened with a complementary set of molecules of claim 1, and,
b) detecting the binding affinity of said complementary set of molecules of claim 1 in the presence or absence of said compound to be tested.

6. A host cell transformed with a nucleic acid sequence coding for muteins of CD14 or parts of CD14 which bind to HBV components, with said host expressing said insert polypeptides or peptides on its surface.

7. Method for identifying compounds interfering with the interaction between CD14 and HBV components comprising the steps of:
a) contacting the compound to be screened with a cellular host of claim 5, and,
b) detecting the formation of a complex between said compound and said CD14 molecule or a mutein or fragment thereof, or,
c) detecting the interference of said compound with the binding of HBV components with said CD14 molecule or a mutein or a fragment thereof.

8. Method of claim 7, wherein said detection step in b) or c) is a quantitative detection step to determine the affinity of binding said compound.

9. Compound identified by a method of claim 7 or 8.

10. Compound according to claim 9, further modified for use in a delivery system.

11. Method for preventing, treating or alleviating inflammatory reactions in patients comprising the delivery of a compound according to any of claims 9 or 10.

12. Use of a cellular host transformed with a nucleic acid sequence coding for CD14 muteins of CD14 or parts of CD14 which bind to HBV components for identifying compounds which interfere with the binding of HBV to its natural receptor.

13. Use of a molecule as defined in claim 1 for preventing, treating or alleviating HBV infection.

14. Use of a molecule as defined in claim 1 for the preparation of a screening assay for identifying useful compounds to prevent, treat or alleviate HBV infection.

15. Use of a molecule as defined in claim 4 for preventing, treating or alleviating inflammatory reactions.

16. A host cell according to claim 6 used as a model system for testing potential drugs which are able to exert at least one of the following functions:
a) modulate the binding or the affinity of binding of HBV components to said host cell,
b) modulate the expression of CD14 or its muteins or its fragments on the surface of said host cell,
c) modulate the activity of HBV components or the activity of CD14 or its muteins or fragments thereof, or
d) interfere with the replication of HBV in said host cell.

17. Mammalian non-human transgenic animal able of being infected by HBV and possibly suffering from hepatocellular damage caused by the consecutive or inducible expression of a nucleic acid encoding a CD14 mutein or a part of CD14 introduced into the genome of said transgenic animal, as a model system for testing potential drugs which are able to exert at least one of the following functions:
a) modulate the binding or the affinity of binding of HBV to a cell of said transgenic animal,
b) modulate the expression of CD14 or its muteins or its fragments on the surface of a cell of said transgenic animal,
c) modulate the activity of HBV components or the activity of CD14 or its muteins or fragments thereof, or
d) interfere with the replication of HBV in said transgenic animal.

18. Method for identifying potential HBV drugs comprising the use of a mammalian non-human transgenic animal wherein the animal CD14 gene is replaced by a human CD14 gene or wherein at least part of a human CD14 gene is introduced.

19. Compound identified by a method of claim 18.

20. Method for the production of a composition comprising the method of any of claims 7, 8 or 18 and possibly mixing the compound identified with a pharmaceutically acceptable carrier.

21. Method for preventing, treating or alleviating HBV infections comprising the delivery of a compound identified by the method of any of claims 7, 8 or 18.
